# EUROPEAN PATENT APPLICATION

(11) **EP 4 523 688 A1**
(43) Date of publication of application: **19.03.2025**
(21) Application number: 23803832.7
(22) Date of filing: 10.05.2023
(51) Int. Cl.: A61K 31/496, A61K 31/519, A61K 45/06, A61P 35/00, A23L 33/10

(54) **COMPOSITION CONTAINING ARIPIPRAZOLE AS ACTIVE INGREDIENT FOR ENHANCING ANTICANCER EFFECT OF KRAS G12C GENETIC MUTATION THERAPEUTIC AGENT**

(30) Priority: 10.05.2022 KR 20220057025; 03.05.2023 KR 20230057626
(71) Applicant: Korea Institute of Radiological & Medical Sciences, Seoul 01812 (KR)
(72) Inventor: LIM, Young Bin, Seoul 01745 (KR); LEE, Hae June, Seoul 04978 (KR); KIM, Kwang Seok, Seoul 01747 (KR); LEE, Soo Ho, Daegu 41522 (KR)
(74) Representative: Mewburn Ellis LLP
(86) International application number: PCT/KR2023/006331
(87) International publication number: WO 2023/219409

(57) **Abstract**

The present invention relates to a composition containing aripiprazole as an active ingredient for enhancing the anti-cancer effect of a KRAS G12C genetic mutation therapeutic agent. More specifically, the present invention concerns a composition for enhancing the anticancer effect of a KRAS G12C genetic mutation therapeutic agent, which acts as an enhancer for the anti-cancer effect of the KRAS G12C genetic mutation therapeutic agent when used in combination therapy with aripiprazole, thereby enabling the treatment of cancer. When used at an effective dose in combination with a KRAS G12C genetic mutation therapeutic agent, aripiprazole according to the present invention excellently enhances the anticancer effect of the KRAS G12C genetic mutation therapeutic agent by inducing cancer cell death, etc. Thus, it can be advantageously used as an enhancer for the anti-cancer effect of the KRAS G12C genetic mutation therapeutic agent.

## Description

### Technical Field

The present disclosure relates to a composition for enhancing an anticancer effect of a therapeutic agent for KRAS G12C genetic mutation including aripiprazole as an active ingredient, and more specifically, to a composition for enhancing an anticancer effect of a therapeutic agent for KRAS G12C genetic mutation, which is capable of treating a cancer by acting as an anticancer effect enhancer of a therapeutic agent for KRAS G12C genetic mutation in co-treatment with aripiprazole.

### Background Art

KRAS G12C mutations are involved in cancer development by activation of mutation from glycine to cysteine, occurring at the 12^{th} codon of a KRAS gene. It is known that brain metastasis occurs in about 40% of patients with KRAS G12C-mutated non-small cell lung cancer, and the overall prognosis is poor for patients with brain metastasis. Sotorasib, a therapeutic agent for KRAS G12C genetic mutations, is currently in clinical trials for non-small cell lung cancer, colorectal cancer, and other solid tumors in combination with chemotherapy or PD-1, PD-L1, MEK, EGFR, or SHP2 inhibitors, while adagrasib, a therapeutic agent for KRAS G12C genetic mutation, is undergoing clinical trials for non-small cell lung, colorectal cancer, and other solid tumors in combination with inhibitors of PD-1, EGFR, SHP2, CDK4/6, and or SOS1.

A BH3-only protein is capable of promoting or inducing apoptosis by inhibiting apoptosis regulatory functions. Loss of ERK1/2 activity suppresses phosphorylation of BIM, one of the BH3-only proteins, and loss of ERK-mediated phosphorylation causes stabilization of BIM and an increase in BIM expression. However, BIM induction alone is not sufficient to cause a large amount of apoptosis, as the BIM is bound and inhibited by antiapoptotic proteins, such as those in the BCL2 family. Therefore, BH3 mimetics or BH3-only protein-inducing drugs capable of inhibiting the function of antiapoptotic proteins cause potent apoptosis under concomitant conditions with drugs that induce loss of ERK1/2 activity, and BIM induction by ERK signal inhibition functions as a cause of increased apoptosis.

Using the same principle, BH3 mimetics and BH3-only protein-inducing drugs are being studied to enhance the potent anticancer effects involved with apoptosis under the concomitant conditions with therapeutic agents for KRAS G12C genetic mutation.

### Disclosure of the Invention

### Technical Goals

An object of the present disclosure is to provide a pharmaceutical composition for enhancing an anticancer effect of a therapeutic agent for KRAS G12C genetic mutation, including aripiprazole as an active ingredient.

Another object of the present disclosure is to provide a health functional food composition for enhancing an anticancer effect of a therapeutic agent for KRAS G12C genetic mutation, including aripiprazole as an active ingredient.

Another object of the present disclosure is to provide a pharmaceutical composition for preventing or treating a cancer, including a therapeutic agent for KRAS G12C genetic mutation or a pharmaceutically acceptable salt thereof; and aripiprazole or a pharmaceutically acceptable salt thereof as active ingredients.

Another object of the present disclosure is to provide a health functional food composition for preventing or ameliorating a cancer, including a therapeutic agent for KRAS G12C genetic mutation or a pharmaceutically acceptable salt thereof; and aripiprazole or a pharmaceutically acceptable salt thereof as active ingredients.

### Technical Solutions

To achieve the above objects, the present disclosure provides a pharmaceutical composition for enhancing an anticancer effect of a therapeutic agent for KRAS G12C genetic mutation, including aripiprazole as an active ingredient.

In addition, the present disclosure provides a health functional food composition for enhancing an anticancer effect of a therapeutic agent for KRAS G12C genetic mutation, including aripiprazole as an active ingredient.

In addition, the present disclosure provides a pharmaceutical composition for preventing or treating a cancer, including a therapeutic agent for KRAS G12C genetic mutation or a pharmaceutically acceptable salt thereof; and aripiprazole or a pharmaceutically acceptable salt thereof as active ingredients.

In addition, the present disclosure provides a health functional food composition for preventing or ameliorating a cancer, including a therapeutic agent for KRAS G12C genetic mutation or a pharmaceutically acceptable salt thereof; and aripiprazole or a pharmaceutically acceptable salt thereof as active ingredients.

### Advantageous Effects

The present disclosure relates to a composition for enhancing an anticancer effect of a therapeutic agent for KRAS G12C genetic mutation including aripiprazole as an active ingredient, and more specifically, to a composition for enhancing an anticancer effect of a therapeutic agent for KRAS G12C genetic mutation, which is capable of treating a cancer by acting as an anticancer effect enhancer of a therapeutic agent for KRAS G12C genetic mutation in co-treatment with aripiprazole. By co-administering an effective amount of aripiprazole according to the present disclosure with the therapeutic agent for KRAS G12C genetic mutation, owing to excellent effect in anticancer effect enhancement of the therapeutic agent for KRAS G12C genetic mutation, such as induction of apoptosis of cancer cells, the composition may be useful as an anticancer effect enhancer of the therapeutic agent for KRAS G12C genetic mutation.

### Brief Description of Drawings

FIG. 1 shows results of identifying an effect of aripiprazole treatment on NOXA protein expression in MIA PaCa-2 cells and NCI-H1373 cells.
FIG. 2 shows a schematic diagram to illustrate an effect of expression of NOXA, one of BH3-only proteins, on an anticancer efficacy of a therapeutic agent for KRAS G12C genetic mutation.
FIG. 3 shows results of identifying an effect of treatment of aripiprazole alone or in combination with a therapeutic agent for KRAS G12C genetic mutation on PARP cleavage in MIA PaCa-2 cells.
FIG. 4 shows results of identifying an effect of treatment of aripiprazole alone or in combination with a therapeutic agent for KRAS G12C genetic mutation on PARP cleavage in NCI-H1373 cells.

### Best Mode for Carrying Out the Invention

Hereinafter, the present disclosure will be described in more detail.

The present disclosure provides a pharmaceutical composition for enhancing an anticancer effect of a therapeutic agent for KRAS G12C genetic mutation, including aripiprazole as an active ingredient.

The therapeutic agent for KRAS G12C genetic mutation may be any one selected from the group consisting of sotorasib, adagrasib, GDC-6036, and JDQ-443, but is not limited thereto.

The cancer may be melanoma, breast cancer, lymphoma, brain tumor, brain cancer, colorectal cancer, prostate cancer, lung cancer, osteosarcoma, bone cancer, pancreatic cancer, skin cancer, oral cavity cancer, oropharyngeal cancer, uterine cancer, ovarian cancer, rectal cancer, stomach cancer, endometrial cancer, cervical cancer, vaginal cancer, small intestine cancer, thyroid cancer, parathyroid cancer, chronic or acute leukemia, bladder cancer, kidney cancer, or liver cancer, but is not limited thereto.

The pharmaceutical composition may induce PARP cleavage of cancer cells or increase NOVA expression.

In another embodiment of the present disclosure, the pharmaceutical composition may further include one or more additives selected from the group consisting of suitable carriers, excipients, disintegrators, sweeteners, coating agents, leavening agents, lubricants, glydents, flavoring agents, antioxidants, buffers, bacteriostatic agents, diluents, dispersants, surfactants, binders, and lubricants that are conventionally used in the manufacture of pharmaceutical compositions. Specifically, as for carriers, excipients and diluents, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil may be used, solid preparations for oral administration may include tablets, pills, acids, granules, and capsules, and these solid preparations may be prepared by mixing at least one excipient in the composition, for example, starch, calcium carbonate, sucrose or lactose, and gelatin. In addition to simple excipients, lubricants such as magnesium stearate and talc may also be used. The oral liquid preparations may be suspensions, liquids, emulsions, and syrups and may include various excipients, such as humectants, sweeteners, fragrances, and preservatives, in addition to the simple diluents that are commonly used, such as water and liquid paraffin. Preparations for parenteral administration include sterile aqueous solutions, non-aqueous solvents, suspensions, emulsions, freeze drying agents, and suppositories. As for non-aqueous solvents and suspensions, propylene glycol, polyethylene glycol, vegetable oils such as olive oil, and injectable esters such as ethyl oleate may be used. Witepsol, macrogol, tween 61, cacao butter, laurin fat, and glycerogelatin may be used as base materials of suppositories. According to one embodiment of the present disclosure, the pharmaceutical composition may be administered to a subject in a conventional manner through intravenous, intraarterial, intraperitoneal, intramuscular, intraarterial, intraperitoneal, intrasternal, percutaneous, nasal, inhaled, topical, rectal, oral, intraocular or intracutaneous routes. The dosage of the active ingredient according to the present disclosure may vary depending on the condition and weight of the subject, the type and severity of a disease, the form of drugs, the route and duration of administration and may be appropriately selected by those skilled in the art, and the daily dosage may be 0.01 mg/kg to 200 mg/kg, preferably 0.1 mg/kg to 200 mg/kg, and more preferably 0.1 mg/kg to 100 mg/kg. The administration may be conducted once a day or divided into several doses, which does not limit the scope of the present disclosure.

In addition, the present disclosure provides a health functional food composition for enhancing an anticancer effect of a therapeutic agent for KRAS G12C genetic mutation, including aripiprazole as an active ingredient.

The health functional food may contain various nutritional supplements, vitamins, minerals (electrolytes), flavor agents such as synthetic flavor agents and natural flavor agents, coloring agents and thickeners (cheese and chocolate), pectic acid and salts thereof, alginate and salts thereof, organic acids, protective colloidal thickeners, pH regulators, stabilizers, preservatives, glycerin, alcohol, and carbonating agents used in carbonated beverages. In addition, it may contain pulp for the manufacture of natural fruit juices, synthetic fruit juices, and vegetable drinks. These ingredients may be used independently or in combination. In addition, the health functional food composition may be in the form of any one of meat, sausage, bread, chocolate, candy, snacks, confectionery, pizza, instant noodles, chewing gum, ice cream, soups, beverages, teas, functional waters, drinks, alcohol, and vitamin complexes. In addition, the health functional food may further include food additives, and the suitability as the "food additive" is determined by the standards and criteria related to corresponding items according to the general rules and general test methods of Korean Food Additives Codex approved by the Ministry of Food and Drug Safety, unless otherwise stipulated. The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents. Here, the content of active ingredients added to the food in the process of manufacturing the health functional food may be appropriately adjusted if necessary, and preferably it may be added to be included in an amount of 1 part by weight to 90 parts by weight based on 100 parts by weight of food.

In addition, the present disclosure provides a pharmaceutical composition for preventing or treating a cancer, including a therapeutic agent for KRAS G12C genetic mutation or a pharmaceutically acceptable salt thereof; and aripiprazole or a pharmaceutically acceptable salt thereof as active ingredients.

The therapeutic agent for KRAS G12C genetic mutation may be any one selected from the group consisting of sotorasib, adagrasib, GDC-6036, and JDQ-443, but is not limited thereto.

The cancer may be melanoma, breast cancer, lymphoma, brain tumor, brain cancer, colorectal cancer, prostate cancer, lung cancer, osteosarcoma, bone cancer, pancreatic cancer, skin cancer, oral cavity cancer, oropharyngeal cancer, uterine cancer, ovarian cancer, rectal cancer, stomach cancer, endometrial cancer, cervical cancer, vaginal cancer, small intestine cancer, thyroid cancer, parathyroid cancer, chronic or acute leukemia, bladder cancer, kidney cancer, or liver cancer, but is not limited thereto.

In addition, the present disclosure provides a health functional food composition for preventing or ameliorating a cancer, including a therapeutic agent for KRAS G12C genetic mutation or a pharmaceutically acceptable salt thereof; and aripiprazole or a pharmaceutically acceptable salt thereof as active ingredients.

Hereinafter, the present disclosure will be described in detail through examples to help understanding of the present disclosure. However, the following examples are intended to merely illustrate the content of the present disclosure, but the scope of the present disclosure is not limited to the following examples. Examples of the present disclosure are provided to more completely explain the present disclosure to those with ordinary skill in the art.

### Modes for Carrying Out the Invention

### <Example 1> Identification of an effect of aripiprazole on the expression of a NOXA protein

To identify an effect of aripiprazole on the expression of a NOXA protein in cancer cells, MIA PaCa-2 cells and NCI-H1373 cells were treated with aripiprazole. Eight hours later, proteins were extracted from cells after washing with PBS, and a level of NOXA expression was checked via Western blot.

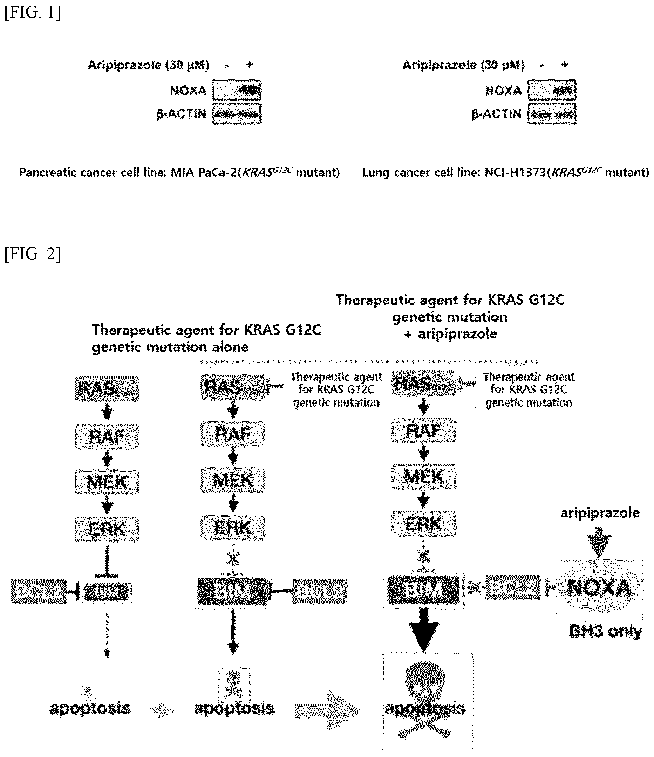

As a result, according to FIG. 1, the expression of NOXA protein increased by aripiprazole treatment, revealing that aripiprazole exhibits an effect of inducing the expression of NOXA protein.

### <Example 2> Identification of an effect of aripiprazole on apoptosis induced by the therapeutic agent for KRAS G12C genetic mutation

### (1) MIA PaCa-2 cells

To identify the effect of aripiprazole on apoptosis induced by the therapeutic agent for KRAS G12C genetic mutation in cancer cells, MIA PaCa-2 cells were treated with aripiprazole alone or in combination with the therapeutic agent for KRAS G12C genetic mutation. Twenty four hours later, proteins were extracted from cells after washing with PBS, and the degree of PARP cleavage was checked via Western blot.

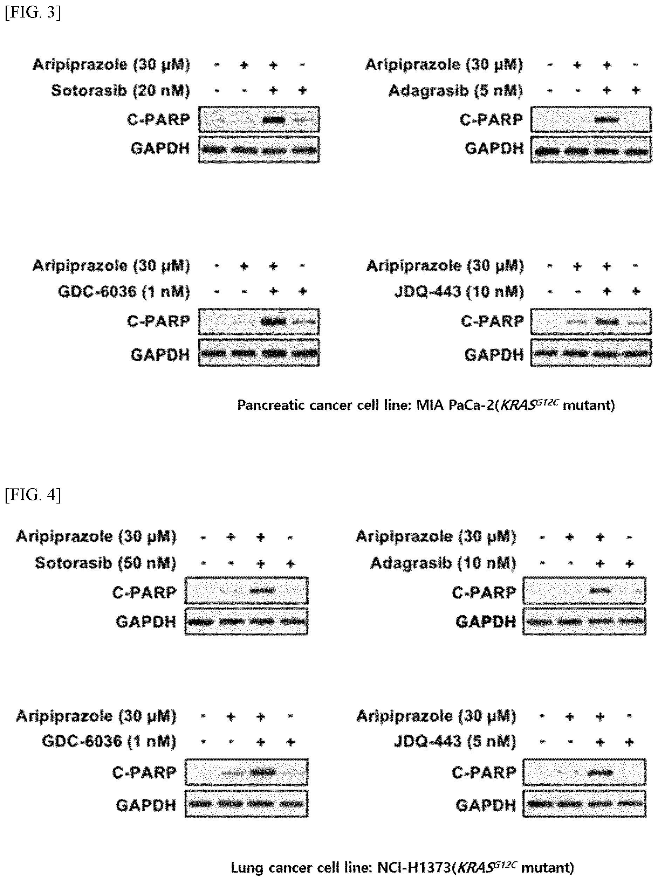

As a result, according to FIG. 3, it was found that the co-treatment of aripiprazole and the therapeutic agent for KRAS G12C genetic mutation showed a superior effect of inducing PARP cleavage than those observed with either aripiprazole or the therapeutic agent for KRAS G12C genetic mutation treated independently.

### (2) NCI-H1373 cells

To identify the effect of aripiprazole on apoptosis induced by the therapeutic agent for KRAS G12C genetic mutation in cancer cells, NCI-H1373 cells were treated with aripiprazole alone or in combination with the therapeutic agent for KRAS G12C genetic mutation. Twenty four hours later, proteins were extracted from cells after washing with PBS, and the degree of PARP cleavage was checked via Western blot.

As a result, according to FIG. 4, it was found that the co-treatment of aripiprazole and the therapeutic agent for KRAS G12C genetic mutation showed a superior effect of inducing PARP cleavage than those observed with either aripiprazole or the therapeutic agent for KRAS G12C genetic mutation treated independently.

The foregoing description of the present disclosure is for illustrative purposes only, and those with ordinary skill in the art to which the present disclosure pertains will understand that it may easily be transformed into other concrete forms without altering the technical idea or essential features of the present disclosure. Therefore, it should be understood that the embodiments described above are illustrative in all respects and not limiting.

The scope of the present disclosure is represented by the claims set forth below, and the meaning and scope of the claims and any altered or modified form derived from the concept of equivalence should be construed as being included in the scope of the disclosure.

## Claims

1. A pharmaceutical composition for enhancing an anticancer effect of a therapeutic agent for KRAS G12C genetic mutation, comprising aripiprazole as an active ingredient.

2. The pharmaceutical composition of claim 1, wherein the therapeutic agent for KRAS G12C genetic mutation is any one selected from the group consisting of sotorasib, adagrasib, GDC-6036, and JDQ-443.

3. The pharmaceutical composition of claim 1, wherein the cancer is melanoma, breast cancer, lymphoma, brain tumor, brain cancer, colorectal cancer, prostate cancer, lung cancer, osteosarcoma, bone cancer, pancreatic cancer, skin cancer, oral cavity cancer, oropharyngeal cancer, uterine cancer, ovarian cancer, rectal cancer, stomach cancer, endometrial cancer, cervical cancer, vaginal cancer, small intestine cancer, thyroid cancer, parathyroid cancer, chronic or acute leukemia, bladder cancer, kidney cancer, or liver cancer.

4. The pharmaceutical composition of claim 1, wherein the pharmaceutical composition induces PARP cleavage of cancer cells or increases NOXA expression.

5. A health functional food composition for enhancing an anticancer effect of a therapeutic agent for KRAS G12C genetic mutation, comprising aripiprazole as an active ingredient.

6. A pharmaceutical composition for preventing or treating a cancer, comprising:
a therapeutic agent for KRAS G12C genetic mutation or a pharmaceutically acceptable salt thereof; and aripiprazole or a pharmaceutically acceptable salt thereof as active ingredients.

7. The pharmaceutical composition of claim 6, wherein the therapeutic agent for KRAS G12C genetic mutation is any one selected from the group consisting of sotorasib, adagrasib, GDC-6036, and JDQ-443.

8. The pharmaceutical composition of claim 6, wherein the cancer is melanoma, breast cancer, lymphoma, brain tumor, brain cancer, colorectal cancer, prostate cancer, lung cancer, osteosarcoma, bone cancer, pancreatic cancer, skin cancer, oral cavity cancer, oropharyngeal cancer, uterine cancer, ovarian cancer, rectal cancer, stomach cancer, endometrial cancer, cervical cancer, vaginal cancer, small intestine cancer, thyroid cancer, parathyroid cancer, chronic or acute leukemia, bladder cancer, kidney cancer, or liver cancer.

9. A health functional food composition for preventing or ameliorating a cancer, comprising: a therapeutic agent for KRAS G12C genetic mutation or a pharmaceutically acceptable salt thereof; and aripiprazole or a pharmaceutically acceptable salt thereof as active ingredients.
